# EUROPÄISCHE PATENTSCHRIFT

(11) **EP 2 160 216 B1**
(45) Veröffentlichungstag und Bekanntmachung des Hinweises auf die Patenterteilung: **10.08.2011**
(21) Anmeldenummer: 08758999.0
(22) Anmeldetag: 04.06.2008
(51) Int. Cl.: A61M 5/50, A61M 5/315

(54) **EINWEGSPRITZE MIT WIEDERVERWENDUNGSSCHUTZ**
DISPOSABLE SYRINGE WITH PROTECTION AGAINST REUSE
SERINGUE À USAGE UNIQUE COMPRENANT UNE PROTECTION CONTRE LA RÉUTILISATION

(30) Priorität: 04.06.2007 DE 102007026972
(43) Veröffentlichungstag der Anmeldung: 10.03.2010
(73) Patentinhaber: Cet Consulting Engineering Trading Monika Eccard, 71394 Kernen-Stetten (DE)
(72) Erfinder: ECCARD, Monika, 71394 Kernen (DE)
(74) Vertreter: Bulling, Alexander
(86) Internationale Anmeldenummer: PCT/EP2008/004441
(87) Internationale Veröffentlichungsnummer: WO 2008/148534

(56) Entgegenhaltungen:
- WO-A-89/02759
- WO-A-90/03818
- WO-A-2004/033008
- US-A1- 2006 211 985

## Beschreibung

Die Erfindung betrifft Einwegspritze mit Wiederverwendungsschutz, die einen Zylinder mit einer Spritzendüse und einen Kolben umfasst, der einen der Spritzendüse zugewandten Kolbenabschnitt, eine Kolbenstange und einen dem Kolbenabschnitt abgewandten Betätigungsabschnitt aufweist.

Aus der EP 557 511 A1, der GB 2 246 297 A sowie der EP 442 260 A1 sind derartige Einwegspritzen bekannt, welche im Bereich der Spritzendüse und der Spritzendüse zugewandten Seite des Kolbenabschnitts Rastabschnitte aufweisen, die beim vollständigen Einschieben des Kolbenabschnitts in den Zylinder miteinander verrasten. Eine ebenfalls in diesem vorderen Bereich der Einwegspritze vorhandene Sollbruchstelle gewährleistet die sichere Zerstörung der Spritze nach dem einmaligen Gebrauch. Nachteilig an diesen Einwegspritzen ist, dass die Rastabschnitte sich in einem mit einer zu injizierenden Substanz befüllbaren Bereich der Spritze befinden und somit bei der Benutzung der Spritze einem direkten Kontakt mit der zu injizierenden Substanz ausgesetzt sind. Des Weiteren schränken die Rastabschnitte das nutzbare Volumen des Zylinders ein. Außerdem umfassen die Rastabschnitte der in den beiden erstgenannten Druckschriften beschriebenen Spritzen Hohlräume, welche ein Blindvolumen bilden, welches beim Aufziehen der zu injizierenden Substanz in die Spritze ein Teil dieser Substanz aufnimmt, welcher jedoch beim anschließenden Injizieren in der Spritze verbleibt.

Aus der US 2004/0127859 A1 ist eine Spritze bekannt, die Rastabschnitte aufweist, die beim Injektionsvorgang nicht in Kontakt mit der zu injizierenden Substanz treten. Allerdings zielt die dort beschriebene Erfindung auf die Vermeidung eines Rückflusses der Substanz in die Spritze ab, so dass in dieser Spritze keine Mittel zur Sicherstellung eines einmaligen Gebrauchs vorgesehen sind.

Aus der US2006/211985A1 ist eine weitere Spritze vorbekannt.

Aufgabe der Erfindung ist es, eine Einwegspritze mit Wiederverwendungsschutz zu schaffen, die einfach und kostengünstig herzustellen ist und deren Mittel zur Bereitstellung des Wiederverwendungsschutzes außerhalb des mit einer zu injizierenden Substanz befüllbaren Bereichs der Einwegspritze angeordnet sind. Zudem soll eine einfache und bedienerfreundliche Handhabung der Einwegspritze gesichert werden.

Zur Lösung der Aufgabe wird eine Einwegspritze mit den Merkmalen des Anspruchs 1 und des Anspruchs 2 vorgeschlagen.

Dadurch, dass sich der Rastabschnitt, der Gegenrastabschnitt sowie die Sollbruchstelle außerhalb eines mit einer zu injizierenden Flüssigkeit befüllbaren Bereichs der Einwegspritze befinden, beanspruchen diese kein Volumen des befüllbaren Bereich, wodurch insbesondere bei Einwegspritzen kleinen Durchmessers ein relativ kurzen Zylinder vorgesehen werden kann und eine beispielsweise auf den Zylinder aufgedruckte Skala zur Anzeige des momentanen Volumens des befüllbaren Bereichs linear ausgebildet werden kann. Somit wird die Handhabung dieser Einwegspritze mit Wiederverwendungsschutz erleichtert. Ferner weist die Einwegspritze kein durch den Wiederverwendungsschutz bedingtes Blindvolumen und keine durch einen Rastabschnitt oder eine Sollbruchstelle verursachte Verengung im Bereich der Spritzendüse auf.

Hierbei ist besonders bevorzugt, dass der Rastabschnitt eine in der Kolbenstange vorgesehenen, radial nach innen verlaufenden Schulter umfasst und dass der Gegenrastabschnitt nach innen ragend ausgebildet ist und bei eingeschobener Kolbenstange die Schulter zur Verrastung hintergreift, wobei der Gegenrastabschnitt wenigstens während eines Abschnitts eines Einführwegs vor dem vollständigen Einschieben des Kolbes in den Zylinder derart unter Vorspannung an der Kolbenstange anliegt dass eine in axialer Richtung dem Einschieben entgegenwirkende Kraft entlang des gesamten Einführwegs nicht oder lediglich geringfügig zunimmt. Hierdurch wird zumindest weitgehend vermieden, dass die dem Einschieben entgegenwirkende Kraft, die sich für den Benutzer der Einwegspritze als Widerstand beim Einschieben des Kolbens in den Zylinder bemerkbar macht, sprungartig zunimmt, wenn der Rastabschnitt den Gegenrastabschnitt passiert. Hierdurch wird eine einfache und bedienerfreundliche Handhabung der Einwegspritze gesichert.

Die Einwegspritze kann vorteilhafterweise so ausgelegt werden, dass eine erste Komponente der Einführkraft, die durch das Zusammenwirken des Rastabschnitts mit dem Gegenrastabschnitt verursacht wird, vorzugsweise entlang des gesamten Einführwegs, deutlich kleiner ist als eine zweite Komponente der Einführkraft, die von einer Reibung des Kolbenabschnitts an einer Innenwand des Zylinders herrührt. Der Einfluss des Rastabschnitts bzw. des Gegenrastabschnitts auf die gesamte dem Einschieben entgegenwirkende Kraft wird dadurch reduziert. Eine relative Änderung der Kraft aufgrund des Zusammenwirkens des Rastabschnitts mit dem Gegenrastabschnitt wird somit reduziert und die Handhabung der Einwegspritze weiter erleichtert.

Gemäß Anspruch 1 ist vorgesehen, dass der Gegenrastabschnitt während des wenigstens weitgehend gesamten Einführwegs des Kolbens unter Vorspannung an der Oberfläche der Kolbenstange anliegt. Hierbei kann vorgesehen werden, dass der Kolben an der dem Kolbenabschnitt zugewandten Seite der Schulter einen Abschnitt aufweist, der im Längsschnitt radial nach außen durch parallel zu einer Mittellängsachse des Kolbens verlaufende Kanten begrenzt ist.

Ferner ist gemäß Anspruch 2 vorgesehen, dass der Rastabschnitt während eines Abschnitts des Einführwegs des Kolbens unter Vorspannung am Gegenrastabschnitt anliegt, wobei zur Erzeugung der Vorspannung der Rastabschnitt eine Schräge umfasst, die an einer dem Betätigungsabschnitt zugewandten Seite von der Schulter begrenzt ist, wobei die Schräge eine sich in axialer Richtung erstreckende Länge aufweist, die vorzugsweise das zehnfache bis zwanzigfache einer sich in radialer Richtung erstreckenden Höhe der Schräge beträgt. Hierdurch ergibt sich eine relativ geringe Neigung der Schräge gegenüber der Mittellängsachse des Kolbens, was zu einer vergleichsweise geringen ersten, von dem Zusammenwirken des Rastabschnitts mit dem Gegenrastabschnitt verursachte Komponente der Kraft führt Die Höhe der Schräge entspricht einem Abstand zwischen einer Grundseite der vorzugsweise im Wesentlichen dreieckigen Schnittfläche der Schräge und der von der Mittellängsachse abgewandten Kante der Schulter. Es kann vorgesehen werden, dass Höhe der Schräge einer Breite der Schulter entspricht. Die Schräge bewirkt insbesondere, dass die erste, von dem Zusammenwirken des Rastabschnitts mit dem Gegenrastabschnitt verursachte Komponente der Kraft während des Einschiebens des Kolbens in den Zylinder langsam und kontinuierlich zunimmt, ohne die Handhabung der Einwegspritze zu erschweren. Die Schräge kann derart ausgebildet sein, dass sie sich elastisch verformt, wenn sie unter der Vorspannung steht. Hat die Schräge den Gegenrastabschnitt passiert, dann fällt die Vorspannung im Wesentlichen weg, die Schräge nimmt zumindest weitgehend wieder ihre ursprüngliche Form an, und der Gegenrastabschnitt umgreift die Schulter.

Es ist denkbar, dass die Länge der Schräge mindestens einem Zehntel einer Länge der Kolbenstange mit dem Kolbenabschnitt entspricht.

Ferner ist vorteilhaft, wenn der Gegenrastabschnitt in einem gegenüber einem Hauptzylinderabschnitt des Zylinders einen größeren Innendurchmesser aufweisenden Zylinderabschnitt vorgesehen ist, wobei der Hauptzylinderabschnitt insbesondere zur Aufnahme des zu injizierenden Serums dient. Dadurch kann die Engstelle des Gegenrastabschnitts derart nach radial innen ragend ausgebildet sein, dass die einen Durchmesser aufweist, der in etwa dem Durchmesser des restlichen Zylinders entspricht oder auch geringfügig grösser sein kann. Einher gehen damit eine vereinfachte Bedienung der Spritze sowie fertigungstechnische Vorteile.

Hierbei ist bevorzugt, wenn der Gegenrastabschnitt zur Erzeugung der Vorspannung wenigstens eine Federzunge mit einem radial nach innen ragenden, mit der Schulter zusammenwirkenden Teilabschnitt aufweist, wobei insbesondere die Federzunge in axialer Richtung überstehend an der von der Spritzendüse abgewandten Seite des Zylinders angeordnet ist. Durch die Verwendung von Federzungen kann ein besonders elastischer Gegenrastabschnitt realisiert werden, der solange er nicht verrastet ist, nur mit einer geringen Kraft gegen die Kolbenstange drückt, jedoch in verrastetem Zustand fest in die Schulter der Kolbenstange eingreift und so einem Herausziehen der Kolbenstange entgegenwirkt. Dies ermöglicht die Herstellung besonders leichtgängiger Spritzen mit einem hochwirksamen Rastmechanismus.

Ferner ist denkbar, dass der Rastabschnitt und/oder die Schräge des Ratsabschnitts zur Erzeugung der Vorspannung in radialer und/oder in zur Längsrichtung quer verlaufender Richtung nachgiebig ausgebildet ist.

Es kann auch vorgesehen werden, dass der Rastabschnitt einen wenigstens abschnittsweise umlaufenden radial nach außen weisenden Ringbund und der Gegenrastabschnitt eine wenigstens abschnittsweise radial nach innen weisende Engstelle umfasst, wobei der maximale Außendurchmesser des Ringbundes grösser ist als der minimale Innendurchmesser der Engstelle und die Engstelle des Gegenrastabschnittes wenigstens bedingt elastisch dehnbar ist, so dass der Ringbund beim Einführen der Kolbenstange in den Zylinder die Engstelle passieren kann. Sowohl der Rastabschnitt als auch der Gegenrastabschnitt lassen sich so unter Verwendung der bei der Herstellung von Kunststoffteilen üblicherweise verwendeten Verfahren besonders einfach fertigen. Denn der Ringbund kann auf einfache Weise als eine stellenweise Verdickung der Kolbenstange ausgeführt sein, während die Engstelle ebenso einfach durch eine stellenweise Verdickung der Zylinderwand der Spritze gebildet sein kann. Zur Erhöhung der Elastizität der Engstelle kann darüber hinaus vorgesehen werden, dass der Zylinder Einkerbungen, Verdünnungen oder dergleichen aufweist.

Es kann vorgesehen werden, dass der Rastabschnitt an seiner dem Kolbenabschnitt zugewandten Seite und/oder der Gegenrastabschnitt an seiner von der Spritzendüse abgewandten Seite mindestens eine zu einer Mittellängsachse des Zylinders etwa oder exakt orthogonal angeordnete Anschlagfläche aufweist. Auf diese Weise wird erreicht, dass sobald der Rastabschnitt einmal den Gegenrastabschnitt beim Einschieben der Kolbenstange in den Zylinder passiert hat, ein erneutes Passieren des Rastabschnitts nicht oder nur sehr bei kräftigem Ziehen an der Kolbenstange möglich ist. Einem auf das Einschieben folgenden Herausziehen der Kolbenstange wird also entgegengewirkt.

Darüber hinaus kann vorgesehen werden, dass der Rastabschnitt an seiner dem Kolbenabschnitt zugewandten Seite und/oder der Gegenrastabschnitt an seiner der Spritzendüse abgewandten Seite eine Einführschräge aufweist. Auf diese Weise kann der Rastabschnitt den Gegenrastabschnitt beim Einschieben der Kolbenstange in den Zylinder bereits bei einem relativ leichten Druck auf die Kolbenstange passieren. Das Vorschieben des Ringbundes in die Engstelle wird also erleichtert. Es wird somit ein bei der Handhabung der Spritze während des Injektionsvorgangs stellenweise erhöhter Widerstand beim Eindrücken der Kolbenstange reduziert.

Es kann vorgesehen sein, dass die Sollbruchstelle zwischen Kolbenstange und Betätigungsabschnitt liegt. Dadurch wird beim Ziehen am Betätigungsabschnitt bei verrastetem Rastabschnitt und Gegenrastabschnitt zum Zwecke des Herausziehens der Kolbenstange genau dasjenige Bauteil der Spritze, nämlich der Betätigungsabschnitt, das zum Aufbringen einer zur Überwindung der Haltekraft des Rastabschnitts und Gegenrastabschnitts erforderlichen Zugkraft zwingend vorhanden sein muss, von der restlichen Spritze gelöst. Die Spritze wird dadurch unbrauchbar gemacht und somit jeder Versuch einer erneuten Benutzung der Spritze vereitelt.

Es kann vorgesehen sein, dass die Sollbruchstelle einen verdünnten Bereich und/oder einen Bereich mit mindestens einem Querloch umfasst. Auf diese Weise kann unter Verwendung der üblichen Verfahren zur Herstellung von Kunststoffteilen die Sollbruchstelle besonders einfach und kostengünstig ausgeführt werden.

Es ist besonders bevorzugt, dass die Kolbenstange und/oder der Betätigungsabschnitt miteinander zusammenwirkende, von der Sollbruchstelle gesondert ausgebildete Anlageabschnitte derart aufweist, dass eine Abknicken oder Brechen der Sollbruchstelle beim Eindrücken des Kolbens unterbunden wird. Dadurch, dass die Anlageabschnitte beim Druck auf den Betätigungsabschnitt zum Einschieben der Kolbenstange aufeinander aufliegen und somit zumindest ein Teil der Druckkraft und/oder seitlich wirkender Kräfte aufnehmen, wird die Sollbruchstelle beim Einschieben des Kolbens in den Zylinder stabilisiert, während sie bei einer Zugbelastung dennoch leicht bricht, weil die Anlageabschnitte bei einer Zugbelastung nicht gegeneinander wirken können.

Hierbei ist besonders bevorzugt, dass die Anlageabschnitte eine in etwa oder exakt senkrecht und/oder parallel zur Mittellängsachse des Kolbens verlaufende Fläche aufweisen. Im ersteren Fall können die Anlageabschnitte im Wesentlichen in Längsrichtung der Kolbenstange wirkende Druckkräfte aufnehmen, während sie im letzteren Fall im Wesentlichen senkrecht zur Kolbenstange wirkende Querkräfte aufnehmen können, die bei einer Betätigung der Spritze zum Beispiel während einer Injektion auftreten können. Außerdem wird eine beispielsweise von einer Elastizität der Sollbruchstelle herrührende und bei der Betätigung der Spritze auftretende störende Hin und Herbewegung des Betätigungsabschnitts vermieden.

Außerdem kann vorgesehen werden, dass die Anlageabschnitte der Kolbenstange und des Betätigungsabschnitts jeweils konusartig ausgebildet sind und zumindest im Wesentlichen parallel zueinander verlaufen. Dadurch können mit den üblichen Verfahren zur Herstellung von Kunststoffteilen die Auflagen besonders einfach realisiert werden.

Hierbei ist besonders bevorzugt, dass die Anlageabschnitte im Bereich um die Sollbruchstelle vorgesehen sind. Derartig ausgebildete Anlageabschnitte sind besonders wirksam und wirken sich bei der Handhabung der Einwegspritze nicht störend aus. Dennoch sind sie einfach zu realisieren.

Um Sollbruchstellen zu erhalten,, die mit den üblichen Verfahren zur Herstellung von Kunststoffteilen mit einem relativ geringen Aufwand hergestellt werden können, wird darüber hinaus vorgeschlagen, dass der Kolben mindestens zwei als Verbindungsflächen ausgebildete Sollbruchstellen aufweist, die voneinander beabstandet, vorzugsweise innerhalb einer zu der Mittellängsachse des Kolbens orthogonal verlaufenden Ebene, angeordnet sind. Hierdurch ergibt sich außerdem eine gute Belastbarkeit einer Verbindung zwischen dem Betätigungsabschnitt und der Kolbenstange mit seitlich wirkenden Kräften.

Um die Verbindung zwischen dem Betätigungsabschnitt und der Kolbenstange weiter derart zu stabilisieren, dass sie die seitlich wirkende Kräfte aufnehmen kann, wird vorgeschlagen, dass der Kolben eine als eine mittlere Verbindungsfläche ausgebildete Sollbruchstelle an einem mittleren Bereich einer Querschnittsfläche der Kolbenstange aufweist, wobei die mittlere Verbindungsfläche vorzugsweise innerhalb der zu der Mittellängsachse des Kolbens orthogonal verlaufenden Ebene angeordnet ist.

Außerdem kann vorgesehen werden dass ein Abstand zwischen den Sollbruchstellen mindestens die Hälfte eines Radiuses der Kolbenstange beträgt.

Gemäß einer bevorzugten Ausführungsform der vorliegenden Erfindung kann vorgesehen werden, dass die Kolbenstange und/oder der Betätigungsabschnitt zumindest abschnittsweise im Querschnitt aus mehreren, vorzugsweise vier, radial von der Mittellängsachse abragenden, flach ausgebildeten Segmenten gebildet sind. Es kann vorgesehen werden, dass alle benachbarten Segmente mit demselben Winkel gegeneinander geneigt sind. Im Falle von vier Segmenten ergibt sich somit ein kreuzförmiger Querschnitt der Kolbenstange bzw. des Betätigungsabschnitts, wobei die einzelnen Segmente des Kreuzes in einem Winkel von jeweils 90° angeordnet sind. Die Segmente können dabei die Rastabschnitte und insbesondere, falls vorgesehen, die zugehörigen Schrägen umfassen.

Es ist besonders bevorzugt, dass der Kolbenabschnitt, die Kolbenstange, der Betätigungsabschnitt und der Rastabschnitt einstückig miteinander verbunden sind. Auf diese Weise können beispielsweise solche Einwegspritzen vorgesehen werden, die aus nur zwei Bauteilen bestehen. Auf Grund dieser geringen Teilezahl kann die Spritze besonders einfach, schnell sowie unter Anwendung weniger Arbeitsschritte und damit besonders kostengünstig hergestellt werden.

Weitere vorteilhafte Ausgestaltungen und Einzelheiten der Erfindung sind der folgenden Beschreibung zu entnehmen, in der die Erfindung anhand der in der Zeichnung dargestellten Ausführungsbeispiele näher beschrieben und erläutert ist. Dabei zeigen:
- Figur 1 eine geschnittene Seitenansicht einer: erfindungsgemäßen Einwegspritze gemäß einer ersten bevorzugten Ausführungsform mit teilweise eingeschobenen Kolben; Figur 2 eine geschnittene Seitenansicht der Einwegspritze aus Figur 1 mit vollständig eingeschobenen Kolben;
- Figur 2: eine geschnittene Seitenansicht der Einwegspritze aus Figur 1 mit vollständig eingeschobenen Kolben;
- Figur 3: eine geschnittene Seitenansicht eines Kolbens einer erfindungsgemäßen Einwegspritze gemäß einer zweiten bevorzugten Ausführungsform;
- Figur 4: eine geschnittene Seitenansicht einer Einwegspritze gemäß einer dritten bevorzugten Ausführungsform mit unvollständig eingeschobenem Kolben;
- Figur 5: eine Draufsicht auf einen Ausschnitt eines Zylinders einer erfindungsgemäßen Einwegspritze gemäß einer vierten bevorzugten Ausführungsform;
- Figur 6: eine geschnittene Seitenansicht eines Ausschnitts eines Kolbens einer erfindungsgemäßen Einwegspritze gemäß einer fünften bevorzugten Ausführungsform;
- Figur 7: einen Querschnitt des in Figur 6 gezeigten Kolbens entlang einer in Figur 6 gezeigten Schnittlinie;
- Figur 8: eine geschnittene Seitenansicht eines Ausschnitts eines Kolbens einer erfindungsgemäßen Einwegspritze gemäß einer sechsten bevorzugten Ausführungsform;
- Figur 9: einen Querschnitt des in Figur 8 gezeigten Kolbens entlang der in Figur 8 gezeigten Schnittlinie;
- Figur 10: eine geschnittene Seitenansicht eines Ausschnitts eines Kolbens einer erfindungsgemäßen Einwegspritze gemäß einer siebten bevorzugten Ausführungsform;
- Figur 11: eine geschnittene Seitenansicht eines Zylinders einer erfindungsgemäßen Einwegspritze gemäß einer achten bevorzugten Ausführungsform;
- Figur 12: eine geschnittene Seitenansicht eines Kolbens der Einwegspritze aus Figur 11;
- Figur 13: ein Querschnitt des in Figur 12 gezeigten Kolbens entlang der in Figur 12 gezeigten Schnittlinie;
- Figur 14: eine geschnittene Seitenansicht der in den Figuren 11 - 13 gezeigten Einwegspritze bei vollständig in den Zylinder eingeschobenen Kolben; und
- Figur 15: ein Querschnitt ähnlich Figur 13 eines Kolbens einer Einwegspritze gemäß einer neunten bevorzugten Ausführungsform.

In den einzelnen Figuren tragen die gleichen Teile dieselben Bezugszeichen. Die Teile werden in der Regel nur einmal im Detail beschrieben.

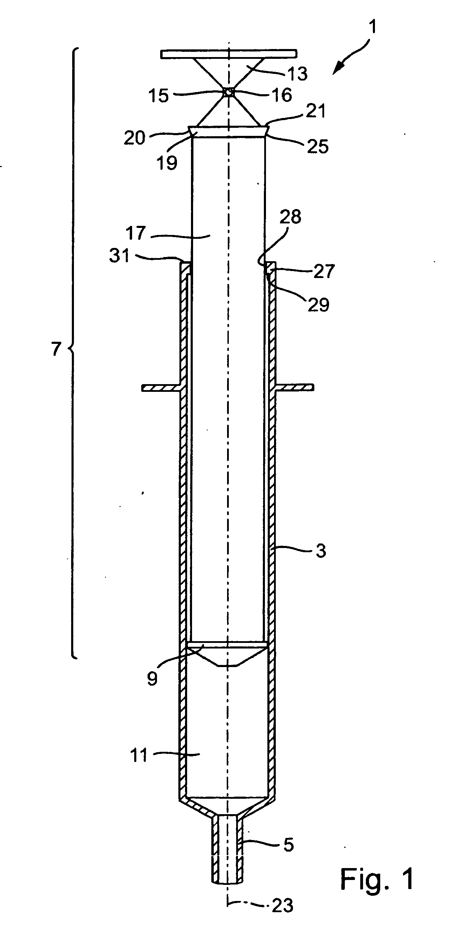

Figur 1 zeigt einen möglichen Aufbau einer erfindungsgemäßen Einwegspritze 1 mit einem Zylinder 3, der an einer Seite eine Spritzendüse 5 aufweist und in dessen von der Spritzendüse 5 abgewandten Seite ein Kolben 7 eingeschoben ist. Der Kolben 7 weist an seiner der Spritzendüse zugewandten Seite einen Kolbenabschnitt 9 auf, der zusammen mit dem Rest des Kolbens 7 im Zylinder 3 hin- und herbewegbar ist. Im Zylinder 3 wird zwischen der Spritzendüse 5 und dem Kolbenabschnitt 9 ein mit einer zu injizierenden Substanz füllbarer Bereich variabler Größe gebildet, der vom Zylinder 3 und dem Kolbenabschnitt 9 begrenzt wird. An seiner dem Kolbenabschnitt 9 entgegengesetzter Seite weist der Kolben 7 einen Betätigungsabschnitt 13 auf, welcher mit einer Sollbruchstelle 15 mit dem Rest des Kolbens 7 verbunden ist.

Die Sollbruchstelle 15 weist ein Querloch 16 auf, das so bemessen ist, dass derjenige Bereich des Querschnitts der Sollbruchstelle 15, der vom Material, aus dem die Sollbruchstelle 15 hergestellt ist, abgedeckt ist, einen derart geringen Flächeninhalt aufweist, dass die Sollbruchstelle beim Anbringen einer Zugkraft bricht, deren Wert einen Wert F_{B} einer Bruchkraft erreicht oder überschreitet.

Zwischen der Sollbruchstelle 15 und dem Kolbenabschnitt 9 weist der Kolben 7 eine Kolbenstange 17 auf, welche ihrerseits einen nach radial außen abstehenden Rastabschnitt 19 aufweist. Der Rastabschnitt 19 weist einen umlaufenden Ringbund 20 asymmetrischen Querschnitts auf, das heißt, der Rastabschnitt 19 weist an seiner dem Betätigungsabschnitt 13 zugewandter Seite eine Anschlagfläche 21 in Form einer zu einer Mittellängsachse 23 des Kolbens 7 orthogonalen, planen und ringförmigen Fläche auf. Der Rastabschnitt 19 umfasst außerdem an seiner der Spritzendüse 5 zugewandten Seite eine Einführschräge 25, welche von einer konischen planen Fläche, die gegenüber der Mittellängsachse 23 des Kolbens 7 geneigt ist, gebildet ist.

Der Zylinder 3 weist an seiner von der Spritzendüse 5 abgewandten Seite einen zum Rastabschnitt 19 komplementären, nach innen ragenden Gegenrastabschnitt 27 auf, welcher eine ringförmige Engstelle 28 begrenzt. An seiner der Spritzendüse 5 zugewandten Seite weist der Gegenrastabschnitt 27 eine kreisringförmige plane Gegenanschlagfläche 29 auf, die mit der Anschlagfläche 21 des Rastabschnitts 19 zusammenwirken kann. Des Weiteren weist der Gegenrastabschnitt 27 an seiner der Spritzendüse 5 abgewandten Seite eine kreisförmige plane Begrenzungsfläche 31 auf, die orthogonal zur Mittellängsachse 23 des Kolbens 7 liegt. Alternativ hierzu kann in einer nicht gezeigten Ausführungsform der Gegenrastabschnitt 27 anstatt der Begrenzungsfläche 31 eine der Einführschräge 25 komplementäre Einführschräge aufweisen, welche ähnlich wie die Einführschräge 25 durch eine konische plane Fläche, die gegenüber der Mittellängsachse 23 des Kolbens 7 geneigt ist, gebildet sein kann.

Der Ablauf einer Benutzung der Einwegspritze 1 ist folgender. Eine einem Benutzer ausgelieferte Einwegspritze 1 befindet sich in einem Zustand, in welchem der Kolben 7 möglichst weit in den Zylinder 3 eingeschoben ist, so dass der füllbare Bereich 11 ein möglichst kleines Volumen aufweist, der Rastabschnitt 19 sich aber ähnlich wie bei dem in Figur 1 gezeigten Zustand der Einwegspritze 1 an der der Spritzendüse 5 abgewandten Seite des Gegenrastabschnitts 27 befindet. Der Benutzer kann dann durch Herausziehen der Kolbenstange 17 samt Kolbenabschnitt 9 durch Ziehen am Betätigungsabschnitt 13 den füllbaren Bereich 11 vergrößern und eine zu injizierende Substanz in die Spritze 1 aufziehen. Um ein Brechen der Sollbruchstelle 15 beim Aufziehen der Einwegspritze 1 auszuschließen, ist die Kraft F_{B}, die zu einem Brechen der Sollbruchstelle 15 bei einer Zugbelastung führen würde, deutlich größer als eine Kraft F_{Z}, die zum Aufziehen des Kolbenabschnitts 9 erforderlich ist.

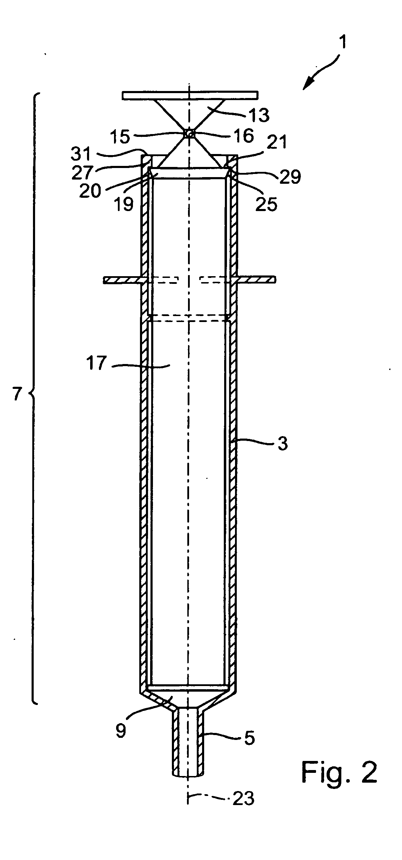

Nach dem Füllen des füllbaren Bereichs 11 der Einwegspritze 1 durch Herausziehen des Kolbens 7 kann der Benutzer die im füllbaren Bereich 11 enthaltene Substanz durch Einschieben des Kolbens 7 durch Druck auf den Betätigungsabschnitt 13 injizieren. Am Ende des Injektionsvorgangs befindet sich die Einwegspritze 1 in der in Figur 2 gezeigten Endstellung, bei welcher der Rastabschnitt 19 den Gegenrastabschnitt 27 passiert hat, so dass die Anschlagfläche 21 gegenüber der Gegenanschlagfläche 29 liegt. Der Rastabschnitt 19 und der Gegenrastabschnitt 27 befinden sich also in einem verrasteten Zustand, was zur Folge hat, dass der Kolben 7 nur dann erneut aus dem Zylinder 3 herausgezogen werden kann, wenn neben der Zugkraft zur Bewegung des Kolbenabschnitts F_{Z} zusätzliche eine dem Herausziehen des Kolbens 7 entgegenwirkende Rastkraft F_{R} der verrasteten Rastabschnitte 19, 27 aufgebracht wird. Eine derart hohe Krafteinleitung in den Kolben 7 wird jedoch dadurch vermieden, dass die maximale von der Sollbruchstelle 15 tolerierte Kraft F_{B} deutlich geringer gewählt ist als die Summe der zum Herausziehen des Kolbenabschnitt erforderlichen Zugkraft F_{Z} und der Rastkraft F_{R}. Versucht der Benutzer also ein zweites Mal, den Kolben 7 der Einwegspritze 1 aus dem Zylinder 3 herauszuziehen, bricht die Sollbruchstelle 15, so dass sich der Betätigungsabschnitt 13 vom Rest des Kolbens 7 löst und die Einwegspritze 1 dadurch unbrauchbar wird.

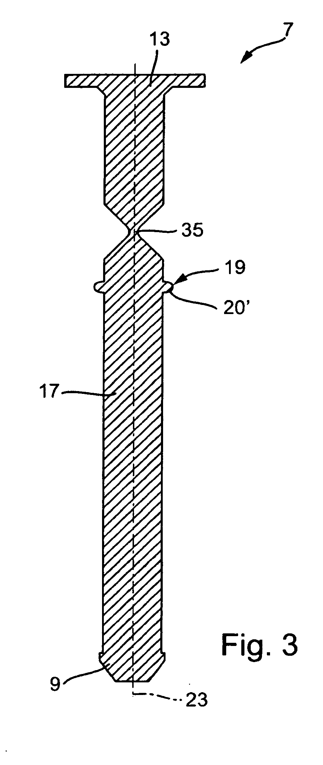

Der Kolben 7 der Einwegspritze 1 kann auch wie in Figur 3 gezeigt ausgeführt werden. Hier weist die Sollbruchstelle 15 anstatt der Querbohrung 16 einen verdünnten Bereich 35 auf, dessen Durchmesser so bemessen ist, dass die Sollbruchstelle 15 beim Anbringen einer Zugkraft, die die Kraft F_{B} erreicht oder überschreitet, bricht. Der Rastabschnitt 19 wird hier von einem Ringbund 20' symmetrischen Querschnitts gebildet, wobei die Oberfläche des Ringbundes 20' gekrümmt ist. Abweichend hiervon kann der Rastabschnitt 19 auch einen asymmetrischen Querschnitt und/oder eine mindestens abschnittsweise plane Oberfläche aufweisen.

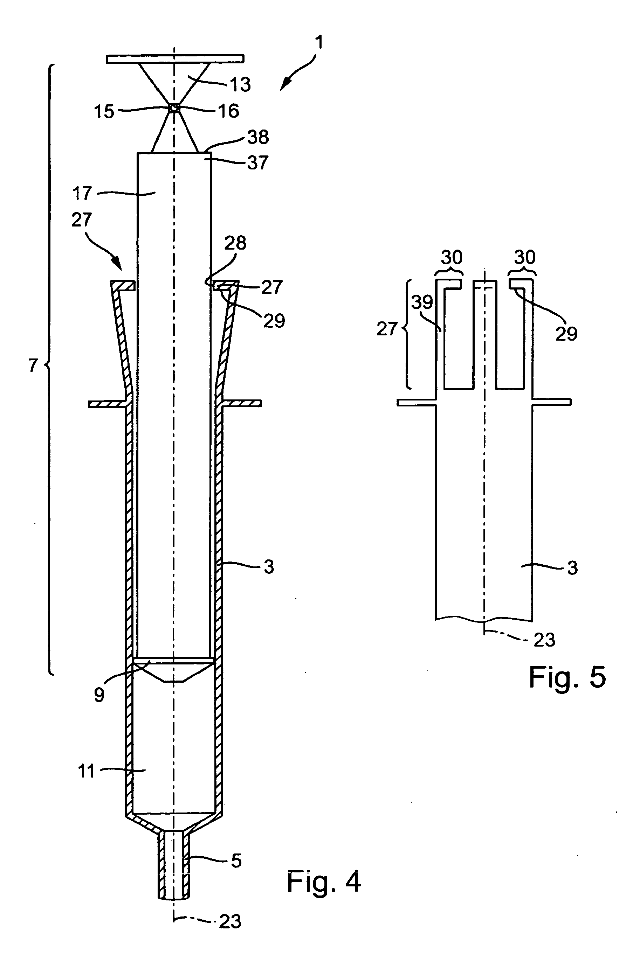

Anstatt eines nach außen ragenden Rastabschnitts 19 kann ein durch eine Aussparung in der Kolbenstange gebildeter Rastabschnitt vorgesehen sein, der eine in Figur 4 gezeigte Schulter 37 aufweist, die eine orthogonal zur Mittellängsachse 23 des Kolbens 7 angeordnete Fläche 38 umfasst. Diese Fläche 38 kann abweichend von der in Figur 4 gezeigten Ausführungsform gegenüber der Mittellängsachse 23 des Kolbens 7 auch geneigt sein oder eine von der in Figur 4 gezeigten planen Form abweichende gekrümmte Form aufweisen.

Der Gegenrastabschnitt 27 ist ebenso wie der Gegenrastabschnitt 27 der in Figur 1 gezeigten

Ausführungsform ringartig nach innen ragend ausgebildet und begrenzt eine Engstelle 28 im Zylinder 3. Jedoch weist der Gegenrastabschnitt 27 hier, solange er nicht verrastet ist, eine Vorspannung auf, das heißt, der Zylinder 3 ist in der Umgebung des Gegenrastabschnitts 27 nach außen hin elastisch aufgeweitet und liegt unter der Vorspannung an der Kolbenstange 17 an.

Im verrasteten Zustand, welcher dann eingenommen wird, wenn die Schulter 37 beim Einschieben des Kolbens 7 in den Zylinder 3 die Gegenanschlagfläche 29 des Gegenrastabschnitts 27 passiert hat, hintergreift die Gegenanschlagfläche 29 des Gegenrastabschnitts 27 die Schulter 37, indem sie sich unter dem Einfluss der Vorspannung radial nach innen bewegt. Einem Zug am Kolben 7 wirkt dann die Rastkraft F_{R} entgegen, so dass ein weiteres Ziehen am Betätigungsabschnitt 13 ebenso wie in der in Figur 1 gezeigten Ausführungsform zu einem Brechen der Sollbruchstelle 15 und somit zu einer Zerstörung der Einwegspritze 1 führt.

In einer nicht gezeigten Ausführungsform weist der Zylinder 3 der Einwegspritze 1 am Gegenrastabschnitt 27 ein oder mehrere Abschnitte auf, in welchen die Wandstärke des Zylinders 3 verringert ist. Dadurch wird die Elastizität des Gegenrastabschnitts 27 erhöht, und eine radial zur Mittellängsachse 23 des Kolbens 7 wirkende Andruckkraft des Gegenrastabschnitts 27 gegen die Kolbenstange 17 wird verringert, was den Bau besonders leichtgängiger Einwegspritzen 1 erlaubt. Anstatt der Bereiche geringerer Wandstärke können am Zylinder 3 auch Einkerbungen, Aussparungen oder ähnliches vorgesehen werden.

Ferner kann in dieser Ausführungsform auch eine Sollbruchstelle 15, die anstatt des Querlochs 16 den verdünnten Bereich 35 aufweist, vorgesehen werden.

Eine weitere Möglichkeit zur Schaffung eines Gegenrastabschnitts 27 höherer Elastizität zeigt die in Figur 5 dargestellte Ausführungsform, in welcher vier Federzungen 39 mit nach innen ragenden Teilabschnitten 30 den Rastabschnitt 27 bilden, welche in axialer Richtung über die der Spritzendüse 5 abgewandte Seite des Zylinders 3 herausragen. Je nach Eigenschaft des zum Herstellen der Einwegspritze 1 verwendeten Materials und den Abmessungen der Einwegspritze 1 kann es sinnvoll oder erforderlich sein, eine abweichende Anzahl von Federzungen 39 vorzusehen oder die Geometrie, insbesondere die Länge der Federzungen 39, anzupassen.

Ist in einem solchen Zylinder 3 einer Einwegspritze 1 der in Figur 4 gezeigte Kolben 7 mit Schulter 37 angebracht, so sind die Federzungen 39 solange der Rastabschnitt 19 und der Gegenrastabschnitt 27 nicht miteinander verrastet sind, radial zur Mittellängsachse 23 des Kolbens 7 elastisch nach außen gebogen. Sobald die Schulter 37 beim Einschieben des Kolbens 7 in den Zylinder 3 die Gegenanschlagfläche 29 des Gegenrastabschnitts 27 passiert hat, bewegen sich die Teilabschnitte 30 aufgrund der von den Federzungen 39 erzeugten Federkraft nach radial innen, so dass die Gegenanschlagfläche 29 des jeweiligen Teilabschnitts 30 die Schulter 37 hintergreift und der Rastabschnitt 19 und der Gegenrastabschnitt 27 somit verrasten und einem Zurückziehen des Kolbens 7 aus dem Zylinder 3 entgegenwirken.

In der in Figur 5 gezeigten Ausführungsform weist der Gegenrastabschnitt 27 mehrere nach innen weisende Teilabschnitte 30 auf, während die Gegenrastabschnitte 27 der in den Figuren 1 bis 4 beschriebenen Ausführungsformen eine einzige den ganzen Umfang des Zylinders 3 umfassende Engstelle 28 aufweisen. Bei letzteren Ausführungsformen kann jedoch ähnlich wie in der Figur 5 gezeigten Ausführungsform auch vorgesehen werden, dass der Gegenrastabschnitt 27 mehrere segmentartige Teilabschnitte 30 aufweist, die in den Zylinder 3 radial hineinragen.

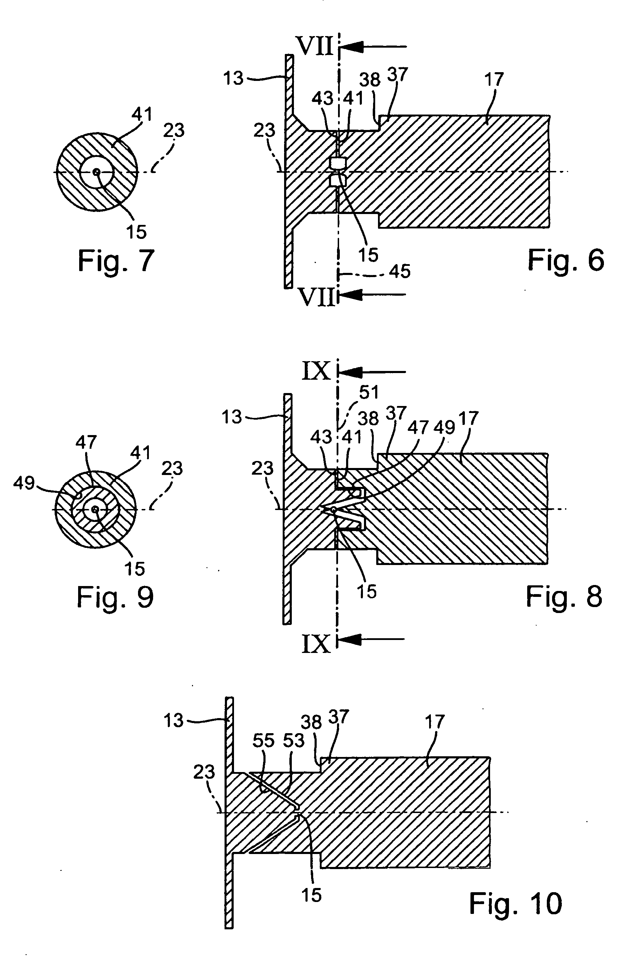

Figur 6 zeigt einen Teil des Kolbens 7 mit Kolbenstange 17, Sollbruchstelle 15 und Betätigungsabschnitt 13, wobei die Kolbenstange 17 einen zur Mittellängsachse 23 des Kolbens 7 orthogonalen Anlageabschnitt 41 und der Betätigungsabschnitt einen zur Mittellängsachse 23 des Kolbens 7 orthogonalen Gegenanlageabschnitt 43 aufweisen. Abweichend von dem in Figur 6 gezeigten planen orthogonalen Anlageabschnitt 41 und Gegenanlageabschnitt 43 können diese auch eine unebene Oberfläche aufweisen. Sie können beispielsweise gezackt oder geriffelt sein. Beim Einschieben des Kolbens 7 in den Zylinder 3 durch Druck auf den Betätigungsabschnitt 13 wirken der orthogonale Anlageabschnitt 41 und der orthogonale Gegenanlageabschnitt 43 gegeneinander und nehmen so mindestens einen Teil der Druckkraft auf, die zum Einschieben des Kolbens 7 benötigt wird und verhindern so ein Abknicken und/oder Brechen der Sollbruchstelle 15 beim Einschieben des Kolbens 7 in den Zylinder 3.

Wie in Figur 7 dargestellt, die einen Schnitt durch die in Figur 6 gezeigte Kolbenstange 17 entlang einer Schnittlinie 45 zeigt, weist der orthogonale Anlageabschnitt 41 die Form einer Scheibe auf. Dementsprechend ist auch der orthogonale Gegenanlageabschnitt 43 als Scheibe ausgebildet. Abweichend hiervon kann der orthogonale Anlageabschnitt 41 und/oder der entsprechende Gegenanlageabschnitt 43 eine abweichende Form aufweisen, insbesondere können sie ein oder mehrere Kreissegmente umfassen.

Außerdem können die Kolbenstangen 17 und der Betätigungsabschnitt 13 jeweils wie in Figur 8 gezeigt einen parallel zur Mittellängsachse 23 des Kolbens 7 längs verlaufende Anlageabschnitt 47 und einen längs verlaufende Gegenanlageabschnitt 49 aufweisen. Sowohl beim Einschieben des Kolbens 7 in den Zylinder 3 als auch beim Herausziehen des Kolbens 7 aus dem Zylinder 3 wirkt der längs verlaufende Anlageabschnitt 47 gegen den entsprechenden Gegenanlageabschnitt 49, um solche Kräfte aufzunehmen, die orthogonal zur Mittellängsachse 23 des Kolbens 7 wirken, um störende Hin- und Herbewegungen des Betätigungsabschnitts 13 oder gar ein Brechen der Sollbruchstelle 15 auf Grund dieser Kräfte zu vermeiden.

Figur 9 zeigt einen Schnitt durch die in Figur 8 gezeigte Anordnung entlang einer Schnittlinie 51. Auch hier umfassen der orthogonale Anlageabschnitt 41, der orthogonale Gegenanlageabschnitt 43, der längs verlaufende Anlageabschnitt 47 und der längs verlaufende Gegenanlageabschnitt 49 den gesamten Umfang des Kolbens 7, jedoch können diese auch so ausgebildet sein, dass sie den Umfang des Kolbens 7 nur abschnittsweise umfassen, also segmentartig ausgebildet sind.

In der in Figur 10 gezeigten Ausführungsform ist ein quer verlaufender Anlageabschnitt 53 und ein quer verlaufender Gegenanlageabschnitt 55 schräg zur Mittellängsachse 23 des Kolbens 7 angeordnet, wobei der quer verlaufende Anlageabschnitte 53 des Betätigungsabschnitts 13 der Kolbenstange 17 konusartig ausgebildet ist und der entsprechende Gegenanlageabschnitt 55 eine kegelartige Gestalt aufweist. Da auf Grund der schrägen Lage der Abschnitte 53, 55 diese längs der Mittellängsachse 23 des Kolbens 7 ausgeübte Druckkräfte zum Einschieben des Kolbens 7 in den Zylinder 3 aufnehmen können, weist die in Figur 10 gezeigte Ausführungsform keinen orthogonalen Anlageabschnitt 41 bzw. Gegenanlageabschnitt 43 auf. Dennoch können diese, um die Druckkräfte noch besser aufnehmen zu können, zusätzlich zu dem quer verlaufenden Anlageabschnitt 53 bzw. dem entsprechenden Gegenanlageabschnitt 55 vorgesehen werden. Um Querkräfte noch besser aufnehmen zu können, können zusätzlich der längs verlaufende Anlageabschnitt 49 und der entsprechende Gegenanlageabschnitt 55 vorgesehen werden.

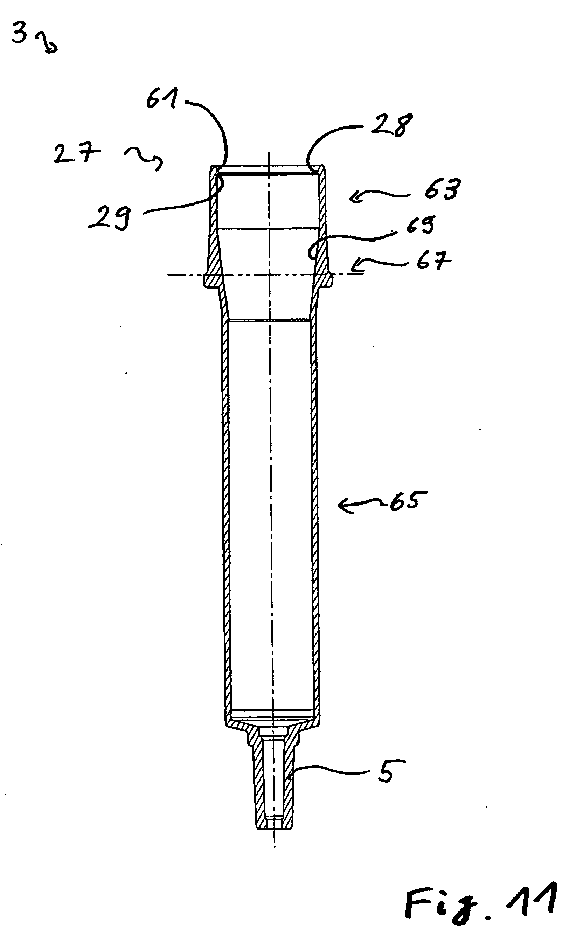

In den Figuren 11 bis 14 ist eine weitere Ausführungsform der vorliegenden Erfindung dargestellt. Der Gegenrastabschnitt 27 des in der Figur 11 gezeigten Zylinders 3 ist an einem von der Spritzendüse 5 abgewandten Ende des Zylinders 3 angeordnet und wird von einer Ringeinschnürung gebildet. Die damit einhergehende ringförmige, den ganzen Umfang des Zylinders 3 umfassende Engstelle 28 ist insbesondere an ihrer der Spritzendüse 5 zugewandten Seite durch die Gegenanschlagfläche 29 und an ihrer von der Spritzendüse 5 abgewandten Seite von einer weiteren Einführschräge 61 begrenzt.

Der Zylinder 3 weist an dem der Spritzendüse 5 abgewandten Ende einen ersten Bereich 63 mit einem erhöhten Innen- und Außendurchmesser auf. Ein der Spritzendüse 5 zugewandter zweiter Bereich 65 des Zylinders 3 weist einen geringeren Innen- und Außendurchmesser auf als der erste Bereich 63 des Zylinders 3. Zwischen dem ersten Bereich 63 und dem zweiten Bereich 65 ist ein Übergangsbereich 67 des Zylinders 3 angeordnet, an dem der Innendurchmesser des Zylinders 3 in Richtung der Spritzendüse 5 kontinuierlich abnimmt. Der Innendurchmesser des Zylinders kann linear abnehmen, sodass eine Innenfläche 69 des Übergangsbereichs 67 eine konische Form aufweist.

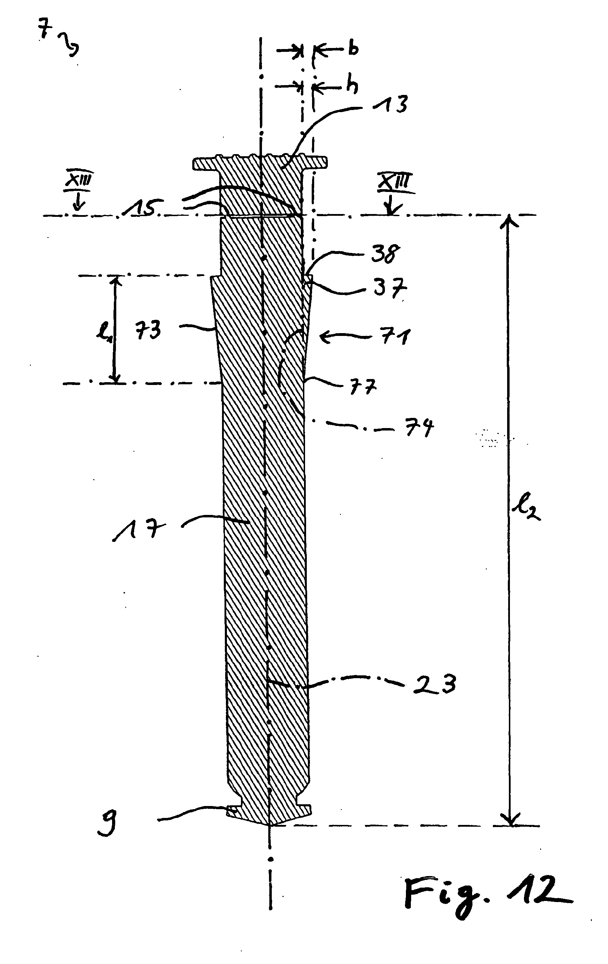

Der in der Figur 12 gezeigte Kolben 7 weist an der Kolbenstange 17 eine lang gestreckte Schräge 71 auf, welche an ihrem dem Betätigungsabschnitt 13 zugewandten Ende durch die zumindest in Wesentlichen orthogonal zur Mittellängsachse 23 angeordneten Fläche 38 der Schulter 37 begrenzt ist. An dieser Schräge 71 nimmt der Außendurchmesser der Kolbenstange 17 in Richtung des Betätigungsabschnitts kontinuierlich zu. Eine Länge l₁ der Schräge 71 beträgt mindestens ein zehntel einer Länge l₂ der Kolbenstange 17 inklusive des Kolbenabschnitts 9. In der gezeigten Ausführungsform beträgt die Länge l₁ der Schräge 71 etwa 17 Prozent der Länge l₂. Die Schräge 71 verläuft weitgehend linear. Die Form der schrägen Oberfläche 73 der Kolbenstange 17 kann der Form der Innenfläche 69 des Übergangsbereichs 67 zumindest weitgehend entsprechen.

Man erkennt anhand der Figur 12, dass die Länge l₁ der Schräge 71 eine Höhe h der Schräge 17 deutlich übersteigt. Die Länge l₁ der Schräge entspricht ungefähr dem zehnfachen der Höhe h der Schräge 71. Die Schräge 71 weist im Längsschnitt des Kolbens 7 eine zumindest im Wesentlichen dreieckige Schnittfläche auf. Die Höhe h der Schräge 71 entspricht einem Abstand zwischen einer parallel zur Mittellängsachse 23 verlaufende Grundseite 74 der Schnittfläche der Schräge 71, und der von der Mittellängsachse 23 abgewandten Kante der Fläche 38. In der gezeigten Ausführungsform entspricht die Höhe h der Schräge 71 einer Breite b der Schulter 37.

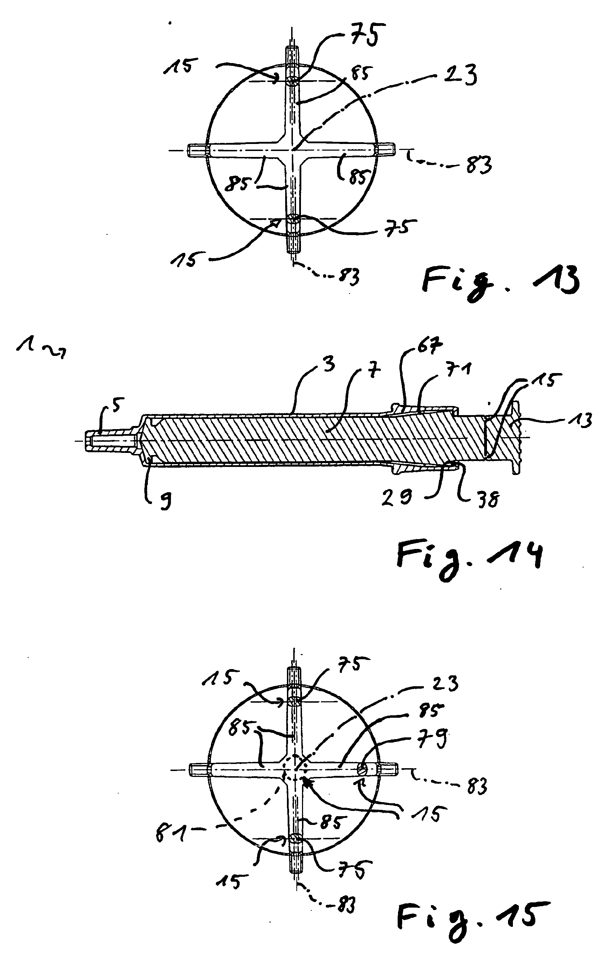

Der Kolben 7 weist zwei Sollbruchstellen 15 auf. Anhand der Figur 13 ist zu erkennen, dass jede Sollbruchstelle 15 durch eine filmartige Verbindungsfläche 75 gebildet ist. Ein Abstand der beiden Verbindungsflächen 75 beträgt mindestens die Hälfte des Außendurchmessers der Kolbenstange 17. In der gezeigten Ausführungsform sind die Verbindungsflächen 75 auf einer zur Mittellängsachse 23 orthogonalen Ebene symmetrisch zur Mittellängsachse 23 angeordnet.

Beim Einschieben des Kolbens 7 in den Zylinder 3 der Einwegspritze 1 passiert zunächst ein unteres Ende 77 der Schräge 71 die Engstelle 28 des Zylinders 3. Anschließend wird - je nach genauer Form und insbesondere in Abhängigkeit von der Elastizität des Kolbens 7 und des Zylinders 3 - die Engstelle 28 nach und nach aufgeweitet und/oder die Kolbenstange 17 zumindest im Bereich der Schräge 71 elastisch verformt. Insbesondere dann, wenn die Kolbenstange, wie weiter unten beschrieben aus sternartig angeordneten Segmenten 85 besteht, kann vorgesehen sein, dass die Kolbenstange, bzw. deren einzelnen Segmenten 85 in Richtung quer zur Längsrichtung weggedrängt werden. Hierbei wird eine radial gegen die Schräge 71 wirkende Vorspannkraft, die die Engstelle 28 gegenüber der Schräge 71 ausübt, aufgebaut. Aufgrund der relativ großen Länge l₁ der Schräge 71 muss hierzu eine vergleichsweise geringe axiale Kraft am Betätigungsabschnitt 13 ausgeübt werden. Da der Außendurchmesser der Schräge 71 in axialer Richtung kontinuierlich und langsam zunimmt, kommt es während des Einschiebens des Kolbens 7 in den Zylinder 3 zu keinem vom Benutzer der Einwegspritze 1 wahrnehmbaren sprungartigem Anstieg eines Widerstands, das heißt einer zum Einschieben des Kolbens 7 in den Zylinder 3 benötigten Kraft.

Da der Kolben 7 der Einwegspritze 1 mehrere voneinander beabstandet angeordnete Verbindungsflächen 75 aufweist, kann eine durch die Verbindungsflächen 75 gebildete Verbindung zwischen dem Betätigungsabschnitt 13 und der Kolbenstange 17 außer der zum Einschieben des Kolbens 7 benötigte axiale Kraft auch seitlich wirkende Kräfte aufnehmen, sodass ein Abknicken des Betätigungsabschnitts 13 vermieden wird.

Hat die Fläche 38 der Schulter 37 die Engstelle 28 passiert, dann verringert sich der Innendurchmesser der Engstelle 28, und die Gegenrastfläche 29 des Zylinders 3 hintergreift die Schulter 37 des Kolbens 7 an der Fläche 38 (siehe Figur 14).

Hat die Gegenrastfläche 29 die Schulter 37 umgriffen, dann ist ein Herausziehen des Kolbens 7 aus dem Zylinder 3 erschwert. Bei einem Versuch, den Kolben 7 am Betätigungsabschnitt 13 wieder aus dem Zylinder 3 herauszuziehen brechen die Sollbruchstellen 15 an den Verbindungsflächen 75.

In einer noch weiteren Ausführungsform ist, wie in der Figur 15 gezeigt, eine weitere Sollbruchstelle 15 in Form einer weiteren Verbindungsfläche 79 vorgesehen. Alle drei Verbindungsflächen 75, 79 sind auf der zur Mittellängsachse 23 orthogonalen Ebene angeordnet. Alle Verbindungsflächen 75, 79 weisen denselben Abstand zu der Mittellängsachse 23 auf. Dieser Abstand beträgt mindestens die Hälfte eines Radiuses der Kolbenstange 17 im Bereich der orthogonalen Ebene.

Darüber hinaus kann auch in einem mittleren Bereich der Kolbenstange 17 eine mittlere Verbindungsfläche 81 angeordnet werden, welche sich ebenfalls in der orthogonalen Ebene befinden kann. Ein Abstand zwischen den durch die Verbindungsflächen 75, 79, 81 gebildeten Sollbruchstellen 15 beträgt in der in der Figur 15 gezeigten Ausführungsform mindestens die Hälfte des Radiuses der Kolbenstange 17 im Bereich der orthogonalen Ebene.

Aus den Figuren 13 und 15 ist ersichtlich, dass die Kolbenstange 17 im Querschnitt kreuzförmig ist. Die obigen Angaben zum Durchmesser und zum Radius der Kolbenstange 17 beziehen sich Längsebenen 83, in denen sich aus der Kreuzform ergebenden Segmente 85 liegen. In der gezeigten Ausführungsform sind vier Segmente 85 vorgesehen. Es kann auch eine hiervon abweichende Anzahl an Segmenten 85 vorgesehen werden.

## Patentansprüche

1. Einwegspritze (1) mit Wiederverwendungsschutz, die einen Zylinder (3) mit einer Spritzendüse (5) und einen Kolben (7) umfasst, der einen der Spritzendüse (5) zugewandten Kolbenabschnitt (9), eine Kolbenstange (17) und einen dem Kolbenabschnitt (9) abgewandten Betätigungsabschnitt (13) aufweist, wobei die Kolbenstange (17) einen Rastabschnitt (19) mit einer in oder an der Kolbenstange (17) vorgesehenen, radial nach innen verlaufenden Schulter (37), der Zylinder (3) einen nach innen ragend ausgebildeten Gegenrastabschnitt (27) und der Kolben (7) mindestens eine Sollbruchstelle (15) derart aufweist, dass bei in den Zylinder (3) eingeschobener Kolbenstange (17) der Gegenrastabschnitt (27) die Schulter (37) zur Verrastung hintergreift und einem Herausziehen der Kolbenstange (17) aus dem Zylinder (3) entgegenwirkt und wobei die Sollbruchstelle (15) so ausgelegt ist, dass sie beim Ziehen am Betätigungsabschnitt (13) bricht, **dadurch gekennzeichnet, dass** die Kolbenstange (17) eine parallel zu einer Mittellängsachse (23) des Kolbens (7) verlaufende Oberfläche aufweist und der Gegenrastabschnitt (27) einstückig mit dem Zylinder (3) verbunden und zumindest abschnittsweise in radialer Richtung nachgiebig ausgebildet ist, sodass der Gegenrastabschnitt (27) während des wenigstens weitgehend gesamten Einführwegs des Kolbens (7) unter Vorspannung an der parallel zur Mittellängsachse (23) verlaufenden Oberfläche der Kolbenstange (17) anliegt.

2. Einwegspritze (1) mit Wiederverwendungsschutz, die einen Zylinder (3) mit einer Spritzendüse (5) und einen Kolben (7) umfasst, der einen der Spritzendüse (5) zugewandten Kolbenabschnitt (9), eine Kolbenstange (17) und einen dem Kolbenabschnitt (9) abgewandten Betätigungsabschnitt (13) aufweist, wobei die Kolbenstange (17) einen Rastabschnitt (19) mit einer in oder an der Kolbenstange (17) vorgesehenen, radial nach innen verlaufenden Schulter (37), der Zylinder (3) einen nach innen ragend ausgebildeten Gegenrastabschnitt (27) und der Kolben (7) mindestens eine Sollbruchstelle (15) derart aufweist, dass bei in den Zylinder (3) eingeschobener Kolbenstange (17) der Gegenrastabschnitt (27) die Schulter (37) zur Verrastung hintergreift und einem Herausziehen der Kolbenstange (17) aus dem Zylinder (3) entgegenwirkt und wobei die Sollbruchstelle (15) so ausgelegt ist, dass sie beim Ziehen am Betätigungsabschnitt (13) bricht, **dadurch gekennzeichnet, dass** der Gegenrastabschnitt (27) einstückig mit dem Zylinder (3) verbunden und zumindest abschnittsweise in radialer Richtung nachgiebig ausgebildet ist, und dass der Rastabschnitt (19) während eines Abschnitts des Einführwegs des Kolbens (17) unter Vorspannung am Gegenrastabschnitt (27) anliegt, wobei der Rastabschnitt (19) zur Erzeugung der Verspannung eine Schräge (71) umfasst, die an einer dem Betätigungsabschnitt (13) zugewandten Seite von der Schulter (37) begrenzt ist, wobei die Schräge (71) eine sich in axialer Richtung erstreckende Länge (l₁) aufweist, die mindestens das zehnfache bis zwanzigfache einer sich in radialer Richtung erstreckenden Höhe (h) der Schräge (71) beträgt.

3. Einwegspritze (1) nach Anspruch 2, **dadurch gekennzeichnet, dass** die Länge (l₁) der Schräge (71) mindestens einem zehntel einer Länge (l₂) der Kolbenstange (17) mit dem Kolbenabschnitt (9) entspricht.

4. Einwegspritze (1) nach Anspruch 2 oder 3, **dadurch gekennzeichnet, dass** der Gegenrastabschnitt (27) in einem gegenüber einem Hauptzylinderabschnitt (65) des Zylinders (3) einen größeren Innendurchmesser aufweisenden Zylinderabschnitt (63) vorgesehen ist.

5. Einwegspritze (1) nach einem der vorhergehenden Ansprüche, **dadurch gekennzeichnet, dass** der Gegenrastabschnitt (27) mehrere segmentartige Teilabschnitte (30) aufweist, die in den Zylinder (3) radial hineinragen.

6. Einwegspritze (1) nach Anspruch 5, **dadurch gekennzeichnet, dass** der Gegenrastabschnitt (27) zur Erzeugung der Vorspannung wenigstens eine sich in axialer Richtung erstreckende und in radialer Richtung nachgiebig ausgebildete Federzunge (39) mit einem radial nach innen ragenden, mit der Schulter (37) zusammenwirkenden Teilabschnitt (30) aufweiset.

7. Einwegspritze (1) nach einem der vorhergehenden Ansprüche, **dadurch gekennzeichnet, dass** die Sollbruchstelle (15) zwischen der Kolbenstange (17) und dem Betätigungsabschnitt (13) liegt.

8. Einwegspritze (1) nach einem der vorhergehenden Ansprüche, **dadurch gekennzeichnet, dass** die Kolbenstange (17) und/oder der Betätigungsabschnitt (13) miteinander zusammenwirkende, von der Sollbruchstelle gesondert ausgebildete Anlageabschnitte (41, 43, 47, 49, 53, 55) derart aufweist, dass eine Abknicken oder Brechen der Sollbruchstelle (15) beim Eindrücken des Kolbens (7) unterbunden wird.

9. Einwegspritze (1) nach Anspruch 8, **dadurch gekennzeichnet, dass** die Anlageabschnitte (41, 43, 47, 49, 53, 55) eine in etwa oder exakt senkrecht und/oder parallel zur Mittellängsachse (23) des Kolbens (7) verlaufende Fläche aufweisen.

10. Einwegspritze (1) nach Anspruch 8 oder 9, **dadurch gekennzeichnet, dass** die Anlageabschnitte (53, 55) der Kolbenstange (17) und des Betätigungsabschnitts (13) jeweils konusartig ausgebildet sind und zumindest im Wesentlichen parallel zueinander verlaufen.

11. Einwegspritze (1) nach einem der Ansprüche 8 bis 10, **dadurch gekennzeichnet, dass** die Anlageabschnitte (41, 43, 47, 49, 53, 55) im Bereich um die Sollbruchstelle (15) vorgesehen sind.

12. Einwegspritze (1) nach einem der vorhergehenden Ansprüche, **dadurch gekennzeichnet, dass** der Kolben (7) mindestens zwei als Verbindungsflächen (75, 79) ausgebildete Sollbruchstellen (15) aufweist, die voneinander beabstandet, vorzugsweise innerhalb einer zu der Mittellängsachse (23) des Kolbens (7) orthogonal verlaufenden Ebene, angeordnet sind.

13. Einwegspritze (1) nach einem der vorhergehenden Ansprüche, **dadurch gekennzeichnet, dass** der Kolben eine als eine mittlere Verbindungsfläche (81) ausgebildete Sollbruchstelle (15) an einem mittleren Bereich einer Querschnittsflache der Kolbenstange (17) aufweist, wobei die mittlere Verbindungsfläche (81) vorzugsweise innerhalb der zu der Mittellängsachse (23) des Kolbens (7) orthogonal verlaufenden Ebene angeordnet ist.

14. Einwegspritze (1) nach einem der vorhergehenden Ansprüche, **dadurch gekennzeichnet, dass** ein Abstand zwischen den Sollbruchstellen (15) mindestens die Hälfte eines Radiuses der Kolbenstange (7) beträgt.

15. Einwegspritze (1) nach einem der vorhergehenden Ansprüche, **dadurch gekennzeichnet, dass** die Kolbenstange (17) und/oder der Betätigungsabschnitt (13) zumindest abschnittsweise im Querschnitt aus mehreren, vorzugsweise vier radial von der Mittellängsachse (23) sternförmig abragenden Segmenten (85) gebildet sind.

16. Einwegspritze (1) nach einem der vorhergehenden Ansprüche, **dadurch gekennzeichnet, dass** der Kolbenabschnitt (9), die Kolbenstange (17), der Betätigungsabschnitt (13) und der Rastabschnitt (19) einstückig miteinander verbunden sind.

## Claims

1. A disposable syringe (1) with protection against reuse, comprising a cylinder (3) with an injection nozzle (5) and a piston (7) that has a piston section (9) facing the injection nozzle (5), a piston rod (17) and an actuation section (13) facing away from the piston section (9), wherein the piston rod (17) has a latching section (19) with a shoulder (37) that is provided in or on the piston rod (17) and runs radially inward, the cylinder (3) has a counter-latching section (27) protruding inward, and the piston (7) has at least one predetermined breaking point (15) in such a way that when the piston rod (17) is introduced into the cylinder (3) the counter-latching section (27) engages behind the shoulder (37) to lock it and counteracts removal the piston rod (17) from the cylinder (3), and wherein the predetermined breaking point (15) is designed such that it breaks when pulling on the actuation section (13), **characterized in that** the piston rod (17) has a surface that runs in parallel to a center longitudinal axis (23) of the piston (7), the cylinder (3) and the counter-latching section (27) are connected to each other in a single piece, and **in that** the counter-latching section (27) is designed to be resilient at least in some sections in the radial direction in such a way that the counter-latching section (27) rests under pretension against the surface of the piston rod (17) during at least most of the insertion path of the piston (7), said surface running in parallel to the center longitudinal axis (23).

2. A disposable syringe (1) with protection against reuse, comprising a cylinder (3) with an injection nozzle (5) and a piston (7) that has a piston section (9) facing the injection nozzle (5), a piston rod (17) and an actuation section (13) facing away from the piston section (9), wherein the piston rod (17) has a latching section (19) with a shoulder (37) that is provided in or on the piston rod (17) and runs radially inward, the cylinder (3) has a counter-latching section (27) protruding inward, and the piston (7) has at least one predetermined breaking point (15) in such a way that when the piston rod (17) is introduced into the cylinder (3) the counter-latching section (27) engages behind the shoulder (37) to lock it and counteracts removal the piston rod (17) from the cylinder(3), and wherein the predetermined breaking point (15) is designed such that it breaks when pulling on the actuation section (13), **characterized in that** the cylinder (3) and the counter-latching section (27) are connected to each other in a single piece, the counter-latching section (27) is designed to be resilient at least in some sections in the radial direction, and **in that** the latching section (19) rests under pretension against the counter-latching section (27) during a section of the insertion path of the piston rod (17), wherein in order to produce the pretension the latching section (19) comprises a chamfer (71) which is bounded by the shoulder (37) on a side facing the actuation section (13), whereby the chamfer (71) has a length (l₁) extending in the axial direction that amounts at least ten to twenty times a height (h) of the chamfer (71) extending in the radial direction.

3. The disposable syringe (1) according to claim 2, **characterized in that** the length (l₁) of the chamfer (71) corresponds to at least one tenth of the length (l₂) of the piston rod (17) with the piston section (9).

4. The disposable syringe (1) according to claim 2 or 3, **characterized in that** the counter-latching (27) section is provided in a cylinder section (63) that has a larger inside diameter compared to a main cylinder section (65) of the cylinder (3).

5. The disposable syringe (1) according to any of the preceding claims, **characterized in that** the counter latching section (27) comprises several segment-like partial sections (30) which protrude radially into the cylinder (3).

6. The disposable syringe (1) according to claim 5, **characterized in that** the counter-latching section (27) comprises at least one flexible tongue (39), which is designed to be resilient in the radial direction and extends in the axial direction, in order to produce the pretension, said tongue having a partial section (30) protruding radially inward and interacting with the shoulder (37).

7. A disposable syringe (1) according to any one of the preceding claims, **characterized in that** the predetermined breaking point (15) is located between the piston rod (17) and the actuation section (13).

8. A disposable syringe (1) according to any one of the preceding claims **characterized in that** the piston rod (17) and/or the actuation section (13) comprise contact sections (41, 43, 47, 49, 53, 55) interacting with each other and designed separately from the predetermined breaking point (15) in such a way that bending or breaking of the predetermined breaking point is prevented when the piston (7) is pressed in.

9. The disposable syringe (1) according to claim 8, **characterized in that** the contact sections (41, 43, 47, 49, 53, 55) have a surface area running approximately or exactly perpendicularly and/or parallel to the center longitudinal axis (23) of the piston (7).

10. The disposable syringe (1) according to claim 8 or 9, **characterized in that** the contact sections (53, 55) of the piston rod (17) and the actuation section (13) are both designed conically and run at least substantially parallel to each other.

11. A disposable syringe (1) according to any one of claims 8 to 10, **characterized in that** contact sections (41, 43, 47, 49, 53, 55) are provided in the area around the predetermined breaking point (15).

12. A disposable syringe (1) according to any one of the preceding claims, **characterized in that** the piston (7) has at least two predetermined breaking points (15) designed as connection surfaces (75, 79) which are disposed at a distance from each other, preferably within a plane running orthogonal to the center longitudinal axis (23) of the piston (7).

13. A disposable syringe (1) according to any one of the preceding claims, **characterized in that** the piston (7) has a predetermined breaking point (15) designed as a center connection surface (81) at a center region of a cross-sectional surface of the piston rod (17), wherein the center connection surface (81) is preferably disposed within the plane running orthogonal to the center longitudinal axis (23) of the piston (7).

14. A disposable syringe (1) according to any one of the preceding claims, **characterized in that** a distance between the predetermined breaking points (15) amounts to at least half of a radius of the piston rod (7).

15. A disposable syringe (1) according to one of the preceding claims, **characterized in that** the piston rod (17) and/or the actuation section (13) at least in some sections of the cross-section are composed of several, preferably four, segments (85) protruding radially in a star shape from the center longitudinal axis (23).

16. A disposable syringe (1) according to one of the preceding claims, **characterized in that** the piston section (9), the piston rod (17), the actuation section (13) and the latching section (19) are connected to each other in a single piece.

## Revendications

1. Seringue à usage unique (1) avec une protection anti-réutilisation, laquelle comporte un cylindre (3) avec une buse d'injection (5) et un piston (7), qui comporte une partie de piston (9), orientée vers la buse d'injection (5), une tige de piston (17) et une partie de manipulation (13), détournée de la partie de piston (9), la tige de piston (17) comportant une partie de blocage (19) avec un épaulement (37) prévu dans ou sur la tige de piston (17) et s'étendant radialement vers l'intérieur, le cylindre (3) comportant une partie de blocage complémentaire (27), réalisée en saillie vers l'intérieur, et le piston (7) comportant au moins une zone destinée à la rupture (15), de telle sorte que, lorsque la tige de piston (17) est insérée dans le cylindre (3), la partie de blocage complémentaire (27) enserre l'épaulement (37) par l'arrière en vue du blocage et s'oppose à une extraction de la tige de piston (17) hors du cylindre (3), et la zone destinée à la rupture (15) étant conçue de telle sorte qu'elle se casse lorsque l'on tire sur la partie de manipulation (13), **caractérisée en ce que** la tige de piston (17) comporte une surface orientée parallèlement à un axe longitudinal médian (23) du piston (7), et la partie de blocage complémentaire (27) est reliée d'un seul tenant au cylindre (3) et est réalisée, au moins par zones, de manière flexible dans la direction radiale, de telle sorte que la partie de blocage complémentaire (27) sur au moins une majeure partie de la trajectoire d'introduction du piston (7) est en appui moyennant une précontrainte contre la surface de la tige de piston (17), qui est orientée parallèlement à l'axe longitudinal médian (23).

2. Seringue à usage unique (1) avec une protection anti-réutilisation, laquelle comporte un cylindre (3) avec une buse d'injection (5) et un piston (7), qui comporte une partie de piston (9), orientée vers la buse d'injection (5), une tige de piston (17) et une partie de manipulation (13), détournée de la partie de piston (9), la tige de piston (17) comportant une partie de blocage (19) avec un épaulement (37) prévu dans ou sur la tige de piston (17) et s'étendant radialement vers l'intérieur, le cylindre (3) comportant une partie de blocage complémentaire (27), réalisée en saillie vers l'intérieur, et le piston (7) comportant au moins une zone destinée à la rupture (15), de telle sorte que, lorsque la tige de piston (17) est insérée dans le cylindre (3), la partie de blocage complémentaire (27) enserre l'épaulement (37) par l'arrière en vue du blocage et s'oppose à une extraction de la tige de piston (17) hors du cylindre (3), et la zone destinée à la rupture (15) étant conçue de telle sorte qu'elle se casse lorsque l'on tire sur la partie de manipulation (13), **caractérisée en ce que** la partie de blocage complémentaire (27) est reliée d'un seul tenant au cylindre (3) et est réalisée, au moins par zones, de manière flexible dans la direction radiale, et **en ce que** la partie de blocage (19), sur un tronçon de la trajectoire d'introduction du piston (7), est en appui moyennant une précontrainte contre la partie de blocage complémentaire (27), la partie de blocage (19) comprenant, en vue de générer la précontrainte, une partie oblique (71) qui, sur un côté orienté vers la partie de manipulation (13), est délimitée par l'épaulement (37), ladite partie oblique (71) ayant une longueur (l₁) orientée dans la direction axiale, qui correspond au moins à dix fois jusqu'à vingt fois à une hauteur (h), orientée dans la direction radiale, de la partie oblique (71).

3. Seringue à usage unique (1) selon la revendication 2, **caractérisée en ce que** la longueur (l₁) de la partie oblique (71) correspond à un dixième de la longueur (l₂) de la tige de piston (17) avec la partie de piston (9).

4. Seringue à usage unique (1) selon la revendication 2 ou 3, **caractérisée en ce que** la partie de blocage complémentaire (27) est prévue dans une partie (63) du cylindre ayant un diamètre intérieur supérieur à celui d'une partie principale (65) du cylindre (3).

5. Seringue à usage unique (1) selon l'une quelconque des revendications précédentes, **caractérisée en ce que** la partie de blocage complémentaire (27) comporte plusieurs tronçons partiels (30) en forme de segments qui s'engagent radialement dans le cylindre (3).

6. Seringue à usage unique (1) selon la revendication 5, **caractérisée en ce que** la partie de blocage complémentaire (27), en vue de générer la précontrainte, comporte au moins une languette à ressort (39), orientée dans la direction axiale et étant flexible dans la direction radiale, munie d'un tronçon partiel (30), s'avançant en saillie radialement vers l'intérieur et coopérant avec l'épaulement (37).

7. Seringue à usage unique (1) selon l'une quelconque des revendications précédentes, **caractérisée en ce que** la zone destinée à la rupture (15) est située entre la tige de piston (17) et la partie de manipulation (13).

8. Seringue à usage unique (1) selon l'une quelconque des revendications précédentes, **caractérisée en ce que** la tige de piston (17) et/ou la partie de manipulation (13) comportent des parties d'appui (41, 43, 47, 49, 53, 55), coopérant entre elles et réalisées séparément de la zone destinée à la rupture (15), de manière à empêcher, pendant l'enfoncement du piston (7), une rupture par pliage ou une fracture de la zone destinée à la rupture (15).

9. Seringue à usage unique (1) selon la revendication 8, **caractérisée en ce que** les parties d'appui (41, 43, 47, 49, 53, 55) comportent une surface qui s'étend sensiblement ou exactement perpendiculairement et/ou parallèlement à l'axe longitudinal médian (23) du piston (7).

10. Seringue à usage unique (1) selon la revendication 8 ou 9, **caractérisée en ce que** les parties d'appui (53, 55) de la tige de piston (17) et de la partie de manipulation (13) sont réalisées chacune en forme de cône et s'étendent au moins sensiblement parallèlement l'une à l'autre.

11. Seringue à usage unique (1) selon l'une quelconque des revendications 8 à 10, **caractérisée en ce que** les parties d'appui (41, 43, 47, 49, 53, 55) sont prévues dans la zone autour de la zone destinée à la rupture (15).

12. Seringue à usage unique (1) selon l'une quelconque des revendications précédentes, **caractérisée en ce que** le piston (7) comporte au moins deux zones destinées à la rupture (15), conçues sous forme de surfaces de liaison (75, 79), lesquelles sont disposées à distance l'une de l'autre, de préférence à l'intérieur d'un plan orienté orthogonalement à l'axe longitudinal médian (23) du piston (7).

13. Seringue à usage unique (1) selon l'une quelconque des revendications précédentes, **caractérisée en ce que** le piston comporte, au niveau d'une zone centrale d'une surface transversale de la tige de piston (17), une zone destinée à la rupture (15) réalisée sous la forme d'une surface de liaison (81) centrale, ladite surface de liaison (81) centrale étant disposée de préférence à l'intérieur du plan orienté orthogonalement à l'axe longitudinal médian (23) du piston (7).

14. Seringue à usage unique (1) selon l'une quelconque des revendications précédentes, **caractérisée en ce qu'**une distance entre les zones destinées à la rupture (15) correspond au moins à la moitié d'un rayon de la tige de piston (17).

15. Seringue à usage unique (1) selon l'une quelconque des revendications précédentes, **caractérisée en ce que** la tige de piston (17) et/ou la partie de manipulation (13) sont formées, au moins par zones dans une coupe transversale, par plusieurs, de préférence quatre segments (85), s'avançant radialement en saillie en forme d'étoile à partir de l'axe longitudinal médian (23).

16. Seringue à usage unique (1) selon l'une quelconque des revendications précédentes, **caractérisée en ce que** la partie de piston (9), la tige de piston (17), la partie de manipulation (13) et la partie de blocage (19) sont reliées d'un seul tenant les unes aux autres.
